# EUROPEAN PATENT SPECIFICATION

(11) **EP 4 443 137 B1**
(45) Date of publication and mention of the grant of the patent: **11.11.2026**
(21) Application number: 23461553.2
(22) Date of filing: 07.04.2023
(51) Int. Cl.: G01N 21/65, G01N 33/50, G01N 33/543

(54) **METHOD FOR IDENTIFYING A NEUTROPHIL-LIKE CELL DERIVED FROM A PROMYELOCYTIC LEUKEMIA CELL**
VERFAHREN ZUR IDENTIFIZIERUNG EINER NEUTROPHIL-ÄHNLICHEN ZELLE, DIE VON EINER PROMYELOZYTISCHEN LEUKÄMIE-ZELLE ABGELEITET WURDE
PROCEDE D'IDENTIFICATION D'UNE CELLULE DE TYPE NEUTROPHILE DERIVEE D'UNE CELLULE DE LEUCEMIE PROMYELOCYTIQUE

(43) Date of publication of application: 09.10.2024
(73) Proprietor: Uniwersytet Jagiellonski, 31-007 Krakow (PL); Uniwersytet Medyczny w Lodzi, 90-419 Lodz (PL)
(72) Inventor: Majzner, Katarzyna, 30-349 Kraków (PL); Adamczyk, Adriana, 30-348 Kraków (PL); Nowakowska, Anna, 31-216 Kraków (PL); Baranska, Malgorzata, 32-087 Trojanowice (PL); Jakubowska, Justyna, 95-050 Konstantynów Lódzki (PL); Mlynarski, Wojciech, 95-010 Anielin Swedowski (PL)
(74) Representative: Witek, Rafal

(56) References cited:
- ZHAO YINGJIA ET AL: "Monitoring the differentiation of dimethyl sulfoxide-induced human leukemia (HL-60) cells by Raman spectroscopy", vol. 52, no. 6, 2 May 2021 (2021-05-02), GB, pages 1086 - 1094, XP093072849, ISSN: 0377-0486, Retrieved from the Internet <URL:https://onlinelibrary.wiley.com/doi/full-xml/10.1002/jrs.6122> DOI: 10.1002/jrs.6122
- CHOI JI SUN ET AL: "Tracing Hematopoietic Progenitor Cell Neutrophilic Differentiation via Raman Spectroscopy", vol. 29, no. 9, 19 September 2018 (2018-09-19), US, pages 3121 - 3128, XP093072854, ISSN: 1043-1802, Retrieved from the Internet <URL:https://www.ncbi.nlm.nih.gov/pmc/articles/PMC6346746/pdf/nihms-1006758.pdf> DOI: 10.1021/acs.bioconjchem.8b00459
- VANNA R ET AL: "Raman spectroscopy for the assessment of acute myeloid leukemia: a proof of concept study", PROGRESS IN BIOMEDICAL OPTICS AND IMAGING, SPIE - INTERNATIONAL SOCIETY FOR OPTICAL ENGINEERING, BELLINGHAM, WA, US, vol. 8939, 4 March 2014 (2014-03-04), pages 89390F - 89390F, XP060033682, ISSN: 1605-7422, ISBN: 978-1-5106-0027-0, DOI: 10.1117/12.2039186

## Description

### Field of the invention

The invention relates to a method of identifying a neutrophil-like cell obtained from a promyelocytic leukaemia cell, in particular, a method of confirming the neutrophil-like differentiation of human promyelocytic leukaemia cell line HL-60, with a PML/RARα rearrangement resulting from a t(15;17) chromosomal translocation. The proposed method is based on calculating the ratio of the integral intensities of Raman bands characteristic for cytochrome, lipids, and MitoBADY reporter, whose signal, togetherwith the integrated band intensities for lipids and cytochrome present in the Raman spectra of living cells, allows the distinction of differentiated and undifferentiated cells. These bands are 750, 2220 and 2850 cm⁻¹ and are attributed to the presence of cytochrome, MitoBADY reporter and lipids respectively.

### State of the art

Neutrophils, also known as neutrophil granulocytes, are the most numerous subpopulation of immune cells circulating in the blood and represent 50-70% of all leukocytes. The process of leukocyte formation (granulocytopoiesis) takes place in the bone marrow, where neutrophils are formed from multipotent stem cells. From the bone marrow, neutrophils enter the circulation, where they live for 6-10 hours, after which they die mainly by apoptosis. Since primary neutrophils present in the bloodstream are short-lived cells, terminally differentiated, and unable to proliferate, any study on their metabolism, signalling pathways, and pathophysiological mechanisms is extremely difficult. An alternative method to research neutrophil physiology is to use a suitable cell line that shows a high similarity to neutrophils. The human HL-60 promyelocytic leukaemia line is such a model. Conducting studies using this model has advantages over studies using primary neutrophils that it can be maintained in culture for an extended period, stably transfected and differentiated into neutrophil-like cells using, for example, dimethyl sulphoxide (DMSO) or retinoic acid. Differentiated HL-60 cells show phenotypes similar to primary neutrophils, such as chemotaxis or phagocytosis. The last 10 years of research on neutrophil biology have shown that these cells are an important component of the immune system and are now an area of great interest to physiologists, haematologists and immunologists alike.Differentiated HL-60 cells exhibit similar phenotypes to primary neutrophil cells, such as chemotaxis and phagocytosis.

The process of differentiation of HL-60 cells into neutrophils is monitored by observing characteristic morphological changes with light microscopy and detecting the expression of myeloid cell surface markers such as CD11b with flow cytometry. In immunophenotyping, CD11b is a leukocyte-specific receptor and is considered a marker for monocyte/macrophages, granulocytes, and natural killer cells. Current standard protocols to identify the differentiation state of immature promyeloblastic cells into mature neutrophils are based on cytometric and molecular studies. The surface marker CD11b is strongly expressed in neutrophils and plays an important role in the function of this group of leukocytes, so it is also commonly used as a marker of HL-60 cell differentiation into neutrophils. However, this approach requires highly specialised equipment and experienced laboratory personnel, with significant costs. In many laboratories that conduct *in vitro* bioassays, the necessary equipment (flow cytometer) and an experienced specialist are unavailable, and qualitative information on cell differentiation is crucial to understand the results obtained or to control ongoing cultures. The ability to differentiate cells *in vitro* into neutrophils allows for designing a cellular model to study neutrophil disorders that can lead to numerous diseases, i.e., immunodeficiencies or leukaemias. Evaluation of differentiated cell content is also a key aspect of the quality control of culture conditions. When cells are cultured for longer than 6 weeks, they may spontaneously begin the differentiation process, which can affect the results of studies using the HL-60 line.

Flow cytometry, although an undeniably rapid and sensitive method, proves to be an extremely expensive technique when utilised for detailed analysis with a wide panel of antibodies. Moreover, the lack of standardization and subjective interpretation of data are significant limitations of this method. Hence, to obtain reliable results, in addition to equipment, experienced and qualified personnel are required to analyse and interpret the results reliably.

Contrary to these techniques is Raman spectroscopy, a method that is rapid and non-destructive for the sample under investigation. It provides complete biochemical information. The cost of purchasing a Raman spectrometer is significantly lower than that of a flow cytometer, while cell preparation is simple and does not require expensive immunofluorescence staining. Furthermore, Raman imaging provides complete information on the biochemical status of the cells to identify additional information on the metabolic and biochemical status of the studied material. With Raman spectroscopy, the use of a simple chemometric tool allows for identifying neutrophil-differentiated cells in a sample of the HL-60 cell line. Moreover, the results obtained with the developed method allow the identification of differentiated cells with a classification sensitivity of 98% and a specificity of 0.96%.

The developed method can be used to verify the stability of the HL-60 model in molecular studies related to leukaemic cell proliferation and differentiation (Collins, 1987) and extracellular vesicle (EV) production studies, including studies to understand the effects of EV produced by leukaemic cells on human stem cells (Lavoie *et al.,* 2018). HL-60 cells, differentiated with 0.8% DMF (dimethylformamide) treatment for 4-5 days, are commonly used as a model of differentiated phagocytic cells and granulocytes in studies of opsonophagocytosis for several vaccines, including *Streptococcus* group B (Guttormsen, Liu and Paoletti, 2008), *Streptococcus pneumoniae* (Romero-Steiner *et al.,* 1997) and *Neisseria meningitidis* (Humphries *et al.,* 2015). Furthermore, cells of the HL-60 line are used as an *in vitro* model for numerous studies of cancer development (Kohram *et al.,* 2018) and the anticancer effects of drugs (Takahashi and Breitman, 1990).

### Summary of the invention

The aim of the invention is to develop a new and rapid method of identifying the differentiation of human promyelocytic leukaemia cell line HL-60 into neutrophils with no need to use cytometric assays.

The subject of the invention is a method of identifying a neutrophil-like cell obtained from a human promyelocytic leukaemia cell as defined in the appended claims.

The invention relates to a novel method of identifying neutrophil-like model cells differentiated from leukaemic cells, which involves measuring the intensity ratio of three bands (750, 2850 and 2220 cm⁻¹) on the Raman spectra of cells incubated with the MitoBADY reporter (IUPAC: triphenyl-[4-(4-phenyl-buta-1,3-diynyl)-benzyl]-phosphonium iodide). These bands are attributed, respectively, to oscillatory vibrations of the porphyrin ring of hemoproteins (750 cm⁻¹), vibrations of the alkyl bond in MitoBADY (2220 cm⁻¹), and C-H stretching vibrations in CH₂ groups in aliphatic lipid chains (2850 cm⁻¹).

The invention can be used to assess the differentiation of HL-60 cells, in a situation where mishandling of the cell culture can result in spontaneous differentiation into neutrophils.

### A detailed description of an embodiment of the invention

The invention is presented in the following embodiments, which however do not constitute a limitation of the claimed protection of the invention.

The embodiment discloses a method to determine the presence of neutrophil-like cells differentiated from leukaemic cells of the HL-60 line, which is concurrently a confirmation of the presence in cells of the t(15;17) chromosomal translocation and the PML/RARα fusion gene (a mutation consisting of the promyelocytic leukaemia gene, PML, located on chromosome 15 and the gene coding for retinoic acid receptor alpha, RARα, which in normal hematopoiesis plays a key role in the differentiation of myeloid progenitor cells) are present in the cells. The study scheme consisted of:
(a) culturing of commercially available HL-60 cell line according to the manufacturer's recommendations and protocols: RPMI 1640 medium (Gibco) supplemented with 10% fetal bovine serum (FBS) (Gibco) and glutaMAX (Gibco). The cells were maintained in an incubator at 37°C, in an atmosphere of 5% CO₂ at a suspension density in the range of (0.3-1.0)·10⁶ cells/ml;
(b) inducing differentiation of HL-60 cells into neutrophil-like cells using all-trans retinoic acid (ATRA), added at a concentration of 1 µM to the culture medium for 48 and 72 h; DMSO, in a total content of 0.001% (v/v), was used as the solvent for ATRA;
(c) preparing substrates made of CaF₂ for Raman measurements by sterilizing them and then coating them with 0.001% poly-L-lysine solution and incubating them at 37°C for 30 min; after this time, the poly-L-lysine solution should be removed and the substrates rinsed twice with distilled water;
(d) after the incubation time of cells with ATRA, sampling 0.5-1 ml of the HL-60 cell suspension and centrifuging (150×g) for 5 min, then removing the supernatant and adding an aliquot of fresh warm culture medium; applying the cells suspended in the fresh medium drop-wise onto the CaF₂ slide coated with poly-L-lysine placed in a Petri dish;
(e) placing the cells for 30 min in an incubator;
(f) gently washing the slide with the cells with PBS solution (without Ca²⁺ and Mg²⁺ ions) to remove the medium and unattached cells;
(g) covering the slides with cells with a solution of 100 nM MitoBADY in HBSS (with Ca²⁺ and Mg²⁺ ions) containing 0.001% DMSO or an analogous HBSS solution without MitoBADY and incubating the cells for 15 min in an incubator;
(h) removing the MitoBADY-containing solution and rinsing the slide once with warm HBSS, then adding fresh HBSS and placing the dish on the piezoelectric table of the Raman spectrometer;
(i) performing Raman measurement of the suspension of living cells in the warm HBSS solution.
(j) calculating Raman integrated intensities of the 750, 2220 and 2850 cm⁻¹ bands;
(k) calculating intensity ratios of the 750/(750+2222) bands (A ratio) and the A/B ratio, where A=750/(750+2222) and B=2850/(2850+2222) for each cell tested;
(l) the corresponding value of the band ratio expressed by A/B and possibly additionally A is an indicator of the presence of a neutrophil-like cell population in the culture.

In a preferred embodiment, the method relates to samples of biological material, which are cells of the HL-60 line (e.g. ECACC, 98070106).

It is particularly preferred, when all-trans retinoic acid (ATRA), known from the state of the art, is used to induce cell differentiation into neutrophils, added to the culture medium for 48 or 72 h at a concentration of 1 µM. It is preferred, when DMSO, in a total content of 0.001% (v/v) in the culture medium, is used as the solvent for ATRA.

It is particularly preferred when the cells are incubated for 15 min in an incubator with an HBSS solution containing 100 nM MitoBADY before assessing the differentiation.

It is preferred when the HBSS solution used contains Ca²⁺ and Mg²⁺ ions.

It is particularly preferred when the concentration of DMSO used in the cell incubation does not exceed 0.001%.

It is particularly preferred when the measurement of the band intensity ratio of 750/(750+2222) (A ratio) and/or the A/B ratio, where A=750/(750+2222) and B=2850/(2850+2222) for the presence of neutrophils is carried out in living cells.

It is preferred when the measurement of the band intensity ratio of 750/(750+2222) (A ratio) and/or the A/B ratio, where A=750/(750+2222) and B=2850/(2850+2222) is performed by analysing the signal from Raman spectra obtained using a confocal Raman imaging system equipped with a 532 nm laser, and the Raman imaging measurement is performed using a water immersion objective. It is preferred to use a Raman imaging microscope as it allows the measurement of multiple spectra from a single cell, thereby tracking and determining any variability within the sample by estimating the standard deviation value.

It is particularly preferred when the cell imaging measurement is carried out for a specimen placed on a substrate that does not exhibit a Raman signal in the spectral range of 700 - 3050 cm⁻¹, such as CaF₂, BaF₂ or ZnSe. It is preferred when the measurement is carried out on slides that increase adherence of the cells to the substrate by coating them with a 0.001% poly-L-lysine solution.

It is equally particularly preferred when the spectra are obtained by excitation of the samples using a laser with a wavelength of 532 nm and a laser power measured in front of the objective in the range of 20-30 mW.

When the value of the A/B ratio, where A=750/(750+2222) and B=2850/(2850+2222) is more than 1.12, the presence of a neutrophil-differentiated cell population is found. In addition, this is confirmed by the value of the band intensity ratio 750/(750+2222) (ratio A) greater than 1.24. The numerical values of the position of the bands given above can vary due to the biological variability of the samples and the spectral resolution of the spectrometer by about 3-5 cm⁻¹.

The advantage of the method of the invention is that it can be used to control the condition of the culture and to evaluate the percentage of neutrophil-differentiated cells in an HL-60 cell culture intended for studies based on cells with the phenotype represented by the HL-60 cell line or for studies based on differentiated cells with a neutrophil phenotype. The invention proposes a quick, simple and relatively inexpensive way to control the degree of cell differentiation. The proposed analysis is based on a rapid and label-free assessment of the content of the reduced form of cytochrome c and lipids relative to the degree of mitochondrial membrane depolarization assessed by the signal intensity of a Raman reporter such as MitoBADY. Another advantage of the solution of the invention is that the protocol can be automated and the determinations can be carried out by people trained to operate the instrument without the need for a specialist in molecular spectroscopy.

### Brief description of the figures

Table 1 evaluation of sensitivity and specificity in identifying neutrophil-differentiated cells using the method underlying the proposed invention;
Fig. 1 presents the comparison of the average spectra of cells incubated with ATRA (Differentiated) and non-incubated with ATRA (Non-differentiated) (living cells) with the indication of the bands 750, 2222 and 2850 cm⁻¹ and the range of their integration (grey background);
Fig. 2 evaluation of the effect of cell biological variability on the ability to identify cells differentiated into neutrophils according to the method proposed in the invention;
Fig. 3 presents the differences in the integrated intensity of the cytochrome (750 cm⁻¹; A), lipids (2850 cm⁻¹; B), MitoBADA (2222 cm⁻¹; C) marker bands, the ratio of the bands 750/(750+2222) (ratio A; D), the ratio 2850/(2850+2222) (ratio B; E), and the quotient of ratios A and B (A/B; F); integrated intensities were calculated for the average spectra of cells (living cells) incubated with ATRA (Differentiated) and non-incubated with ATRA (Non-differentiated).

### Example 1. Use of the method of the invention to monitor the course of HL-60 cell line culture, which was subjected to controlled differentiation into neutrophils

The use of the developed invention involves taking about 0.5-1 ml of cell suspension and centrifugation at 150×g for 5 min, then removing the supernatant and adding fresh medium. The suspension is placed for 30 min at 37°C on a CaF₂ slide pre-coated with 0.001% poly-L-lysine. Coating the CaF₂ slide with poly-L-lysine was performed by incubating it with the solution for 30 min at 37°C and then the poly-L-lysine solution was removed, the slide was washed twice and then dried. After 30 min of incubating the cells on the CaF₂ substrate, the suspension was removed and the substrate was gently rinsed with PBS solution (without Ca²⁺ and Mg²⁺) to remove unattached cells.

The next step requires a 15-minute incubation in a cell incubator of the cells adherent to CaF₂ with a warm 100 nM solution of MitoBADY in HBSS solution (with Ca²⁺ and Mg²⁺ ions) with a maximum DMSO content of 0.001% (v/v).

Prior to Raman imaging, the MitoBADY solution is removed and washed twice with warm HBSS. Finally, a Petri dish with the slide immersed in a warm HBSS solution is placed on the piezoelectric table of a Raman spectrometer equipped with an aqueous objective, and hyperspectral images are obtained with a step of 500 nm and an acquisition time of 0.5 s.

Raman imaging measurements were carried out using a WITec Alpha 300 confocal Raman microscope (Ulm, Germany) equipped with a CCD detector (Andor Technology Ltd). A 63x water immersion objective (Zeiss W Plan-Apochromat 63x, NA=1) was used to measure single cells, which were then immersed in a suspension of cells placed on a substrate made of CaF₂ (25 × 2 mm, Crystran Ltd). Raman spectra were recorded using an excitation laser with a wavelength of 532 nm.

The basic procedure for Raman data pre-processing, used in all Raman measurements, included baseline correction and removal of cosmic rays. For this purpose, functions available in WITec Project Plus 5.1 software (Ulm, Germany) were used. In the first step of the analysis of Raman images, cosmic ray signals recorded by the CCD camera (Cosmic Ray Removal, filter size - 3, dynamic factor - 8) were removed and baseline correction was applied using the appropriate polynomial of degree 3. The final step of the procedure for preparing the spectra for analysis was a smoothing procedure with the Savitzky-Golay algorithm using a polynomial of degree 3 and a window of 7 spectral points. Baseline correction was performed in the fingerprint and CH-stretching regions using a polynomial of degree 3 and in the so-called quiet spectral range (1800-2700 cm⁻¹) using the baseline correction method with circle fitting. After performing the correction, individual spectra of each cell recorded by the imaging method should be separated from the spectra of the buffer, and substrate and then averaged. In Example 1, the k-means cluster analysis (KMCA) available in WITec Project Plus 5.1 software (Ulm, Germany) was performed for this purpose. KMCA was performed using an urban distance calculation (Manhattan) and Ward's clustering algorithm to extract the average spectrum of the entire cell and separate the background from the cell.

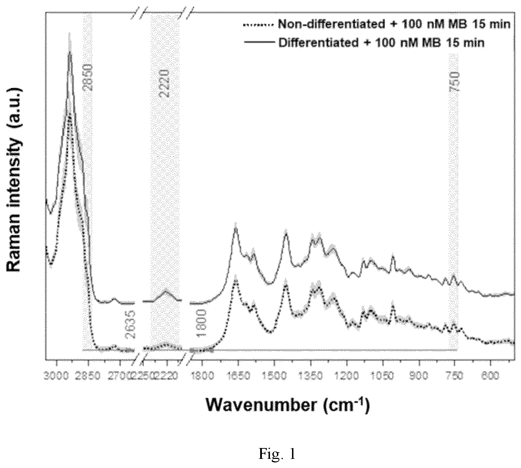

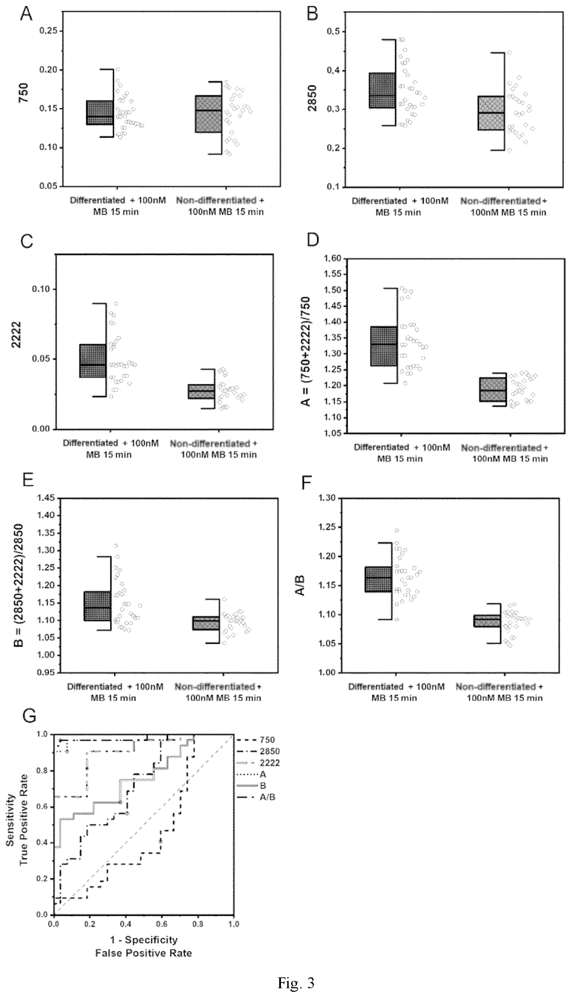

On the spectra of cells incubated with ATRA relative to the spectra of control cells (Fig. 1), an increase in the intensity of the band characteristic of MitoBADY is observed at about 2222 cm⁻¹, among others. However, no change is observed in the intensity of the bands characteristic of cytochrome (e.g. at 750 cm⁻¹), which is a mitochondrial marker. MitoBADY compound is a state-of-the-art Raman reporter known to label mitochondria. The higher band intensity observed for differentiated cells at 2222 cm⁻¹ with no increase in the intensity of cytochrome-specific bands (e.g., the 750 cm⁻¹ band) indicates a different mechanism of cell labelling, which in this case is not sensitive and specific to mitochondria, as known from state of the art, but allows to indirectly identify subtle yet statistically significant changes in mitochondria and lipid metabolism (MitoBADY compound is a lipophilic compound, which is due to its molecular structure). Changes in the ratio of the integrated intensity of the 750 cm⁻¹ (cytochrome), 2220 cm⁻¹ (MitoBADY) and 2850 cm⁻¹ (lipids) bands are evident from the analysis results shown in Fig. 3.

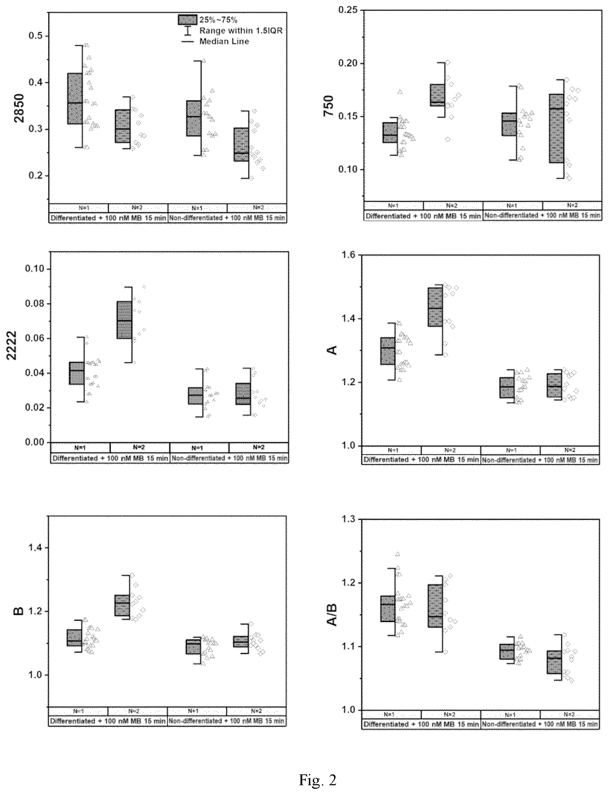

At the same time, it was noted that the ratio of the 750/2850 bands and the integrated intensity of the 2222 cm⁻¹ band alone are not sufficient for the quantitative and sensitive identification of differentiated cells. One of the reasons for this difficulty is the biological variability of cells and differences in the metabolic response of cells to the differentiation process. A comparison of 2 independent experiments on a separately cultured HL-60 cell line, which was subjected to controlled differentiation into neutrophils, is shown in Fig. 2. Despite the variability in the integrated intensity values of the 750 and 2850 bands, the reproducibility of the results obtained with the method described in the application was provided by using normalization based on the MitoBADY signal at 2222 cm⁻¹.

The average spectra of each cell obtained in this way should then be vector normalized in the range of 500-3050 cm⁻¹ and the integration of the 750, 2220 and 2850 cm⁻¹ bands should be performed to calculate the values of their integrated intensities. The positions of the 750, 2220 and 2850 cm⁻¹ bands along with the integration ranges are shown in Fig. 1.

Using the values of the integrated intensities of the 750, 2220 and 2850 cm⁻¹ bands obtained by the integration method, the values of the band intensity ratios A and B should be calculated, where the A ratio denotes the relation: 750/(750+2222), and the B ratio is 2850/(2850+2222). After calculating the A and B ratios, their quotient A/B should be calculated (A/B=(750/(750+2222))/(2850/(2850+2222)).

In Example 1, the A/B ratio of the band integrated intensity calculated from Raman spectra in ATRA-differentiated cell samples is higher than 1.12 (Figs 2 and 3).

The experiments yielded a result - shown in Table 1 and Figs 2 and 3 - indicating that the value of the quantitative band ratio of 750/(750+2222) (A ratio) and/or the A/B ratio, where A=750/(750+2222) and B=2850/(2850+2222) is significantly higher in samples subjected to differentiation into neutrophils with ATRA as compared to the spectra obtained from control cell samples, not subjected to differentiation with ATRA but only incubated with MitoBADY.

### Bibliography:

Collins, S.J. (1987) 'The HL-60 Promyelocytic Leukemia Cell Line: Proliferation, Differentiation, and Cellular Oncogene Expression', Blood, 70(5), pp. 1233-1244. Available at: https://doi.org/https://doi.org/10.1182/blood.V70.5.1233.1233.
Guttormsen, H.-K., Liu, Y. and Paoletti, L.C. (2008) 'Functional activity of antisera to group B streptococcal conjugate vaccines measured with an opsonophagocytosis assay and HL-60 effector cells', Human Vaccines, 4(5), pp. 370-374. Available at: https://doi.org/10.4161/hv.4.5.5988.
Humphries, H.E. et al. (2015) 'Seroprevalence of Antibody-Mediated, Complement-Dependent Opsonophagocytic Activity against Neisseria meningitidis Serogroup B in England', Clinical and vaccine immunology : CVI. 2015/03/04, 22(5), pp. 503-509. Available at: https://doi.org/10.1128/CVI.00100-15.
Kohram, F. et al. (2018) 'Cell type-dependent functions of microRNA-92a.', Journal of cellular biochemistry, 119(7), pp. 5798-5804. Available at: https://doi.org/10.1002/jcb.26765.
Lavoie, J.R. et al. (2018) 'Characterization of leukemia-derived extracellular vesicles and subsequent effects on mesenchymal stem cell phenotype', Cytotherapy, 20(5), p. S22. Available at: https://doi.org/10.1016/j.jcyt.2018.02.050.
Matuszyk, E. et al. (2021) 'Multiplex Raman imaging of organelles in endothelial cells', Spectrochimica Acta Part A: Molecular and Biomolecular Spectroscopy, 255, p. 119658. Available at: https://doi.org/https://doi.org/10.1016/j.saa.2021.119658.
Pieczara, A. et al. (2023) 'Changes in the mitochondrial membrane potential in endothelial cells can be detected by Raman microscopy', Spectrochimica Acta Part A: Molecular and Biomolecular Spectroscopy, 286, p. 121978. Available at: https://doi.org/https://doi.org/10.1016/j.saa.2022.121978.
Romero-Steiner, S. et al. (1997) 'Standardization of an opsonophagocytic assay for the measurement of functional antibody activity against Streptococcus pneumoniae using differentiated HL-60 cells.', Clinical and diagnostic laboratory immunology, 4(4), pp. 415-422. Available at: https://doi.org/10.1128/cdli.4.4.415-422.1997.
Takahashi, N. and Breitman, T.R. (1990) 'Retinoylation of HL-60 proteins. Comparison to labelling by palmitic and myristic acids.', Journal of Biological Chemistry, 265(31), pp. 19158-19162. Available at: https://doi.org/10.1016/S0021-9258(17)30638-5.
Yamakoshi, H. et al. (2015) 'A sensitive and specific Raman probe based on bisarylbutadiyne for live cell imaging of mitochondria', Bioorganic and Medicinal Chemistry Letters, 25(3), pp. 664-667. Available at: https://doi.org/10.1016/j.bmcl.2014.11.080.
Zhao, Y. et al. (2021) "Monitoring the differentiation of dimethyl sulfoxide-induced human leukaemia (HL-60) cells by Raman spectroscopy', Journal of Raman Spectroscopy, n/a(n/a). Available at: https://doi.org/https://doi.org/10.1002/jrs.6122.

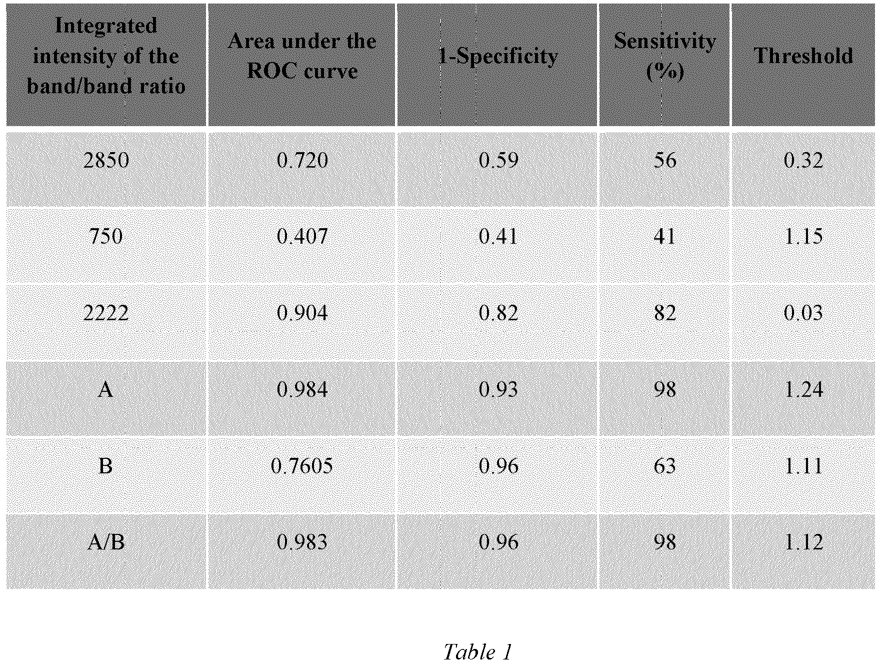

## Claims

1. A method of identifying a neutrophil-like cell obtained from a promyelocytic leukaemia cell, **characterized in that**:
a) the analysed cell is contacted *in vitro* with a MitoBADY reporter, then
b) its Raman spectrum is recorded and the integrated intensities of 750, 2220 and 2850 cm⁻¹ ± 3 cm⁻¹ bands are determined,
c) the values of A and A/B ratios are calculated, where:
A is the ratio of the band integrated intensities 750/(750+2222), while
B is the ratio of the band integrated intensities 2850/(2850+2222),
whereby a cell is considered to be differentiated into a neutrophil when the value of the A/B ratio is greater than 1.12,
whereby preferably the promyelocytic leukaemia cell is human, especially a cell of the HL-60 line.

2. The method of claim 1, is **characterized in that** the cell with a neutrophil-like phenotype is a result of intentional differentiation or a spontaneous process occurring during culture.

3. The method of claim 1 or 2, is **characterized in that** the cell undergoes differentiation into a neutrophil by incubation with all-trans retinoic acid (ATRA) or dimethyl sulphoxide (DMSO).

4. The method of claim 1, is **characterized in that** in step a) a sample is prepared in the form of a suspension containing the cell to be analysed and placed on a substrate made of CaF₂ coated with a layer allowing cell adhesion.

5. The method of claim 1, **characterized in that** in step a) the sample containing the analysed cell is incubated for 15 minutes with a warm solution of 100 nM MitoBADY in Hanks' Balanced Salt Solution (HBSS) solution containing Ca²⁺ and Mg²⁺ ions, with a DMSO content not exceeding 0.001% (v/v).

6. The method of claim 1, **characterized in that** in step b) the sample containing the analysed cell during the measurement of Raman spectra is immersed in a warm HBSS solution containing Ca²⁺ and Mg²⁺ ions.

7. The method of claim 1, is **characterized in that** in step b) the measurement of the Raman spectrum is carried out in living cells.

8. The method of claim 1, is **characterized in that** in step b) the Raman spectrum is recorded with a Raman spectrometer with 532 nm laser excitation, preferably equipped with a water immersion objective.

9. The method of claim 1, **characterized in that** in step (b) the Raman spectrum is recorded from at least 2 different points of the analysed cell showing bands characteristic to cytochrome in the Raman spectra, preferably 750, 1130, 1315 and 1585 cm⁻¹ bands, while the positions of the bands may differ between spectrometers and diffraction gratings used.

10. The method of claim 1, is **characterized in that** in step b) the Raman spectrum is recorded in the fingerprint region (500 - 3050 cm⁻¹), and then the integrated intensities of the 750, 2220 and 2850 cm⁻¹ ± 3 cm⁻¹ bands are calculated.

11. The method of claim 1, **characterized in that** in step c) additionally the presence of the cell with a neutrophil-like phenotype is evidenced by an increase in the intensity of the Raman bands at 2222 cm⁻¹ and an increase in the value of the ratio of the bands' integrated intensities 750/(750+2222) (A ratio), preferably above 1.24.

## Patentansprüche

1. Verfahren zum Identifizieren einer neutrophil-ähnlichen Zelle, die von einer promyelozytischen Leukämie-Zelle erhalten wird, **dadurch gekennzeichnet, dass**:
a) die analysierte Zelle *in vitro* mit einem MitoBADY-Reporter in Kontakt gebracht wird, dann
b) ihr Raman-Spektrum aufgezeichnet wird und die integrierten Intensitäten der Banden bei 750, 2220 und 2850 cm⁻¹ ± 3 cm⁻¹ bestimmt werden,
c) die Werte der Verhältnisse A und A/B berechnet werden, wobei:
A das Verhältnis der integrierten Intensitäten der Bande 750/(750+2222) ist, während
B das Verhältnis der integrierten Intensitäten der Bande 2850/(2850+2222) ist,
wobei eine Zelle als zu einem Neutrophilen differenziert betrachtet wird, wenn der Wert des Verhältnisses A/B größer als 1,12 ist,
wobei die promyelozytische Leukämie-Zelle vorzugsweise menschlich, insbesondere eine Zelle der HL-60-Linie ist.

2. Verfahren nach Anspruch 1, **dadurch gekennzeichnet, dass** die Zelle mit einem neutrophil-ähnlichen Phänotyp das Ergebnis einer absichtlichen Differenzierung oder eines spontanen, während der Kultivierung auftretenden Prozesses ist.

3. Verfahren nach Anspruch 1 oder 2, **dadurch gekennzeichnet, dass** sich die Zelle durch Inkubation mit all-trans-Retinsäure (ATRA) oder Dimethylsulfoxid (DMSO) zu einem Neutrophilen differenziert.

4. Verfahren nach Anspruch 1, **dadurch gekennzeichnet, dass** in Schritt a) eine Probe in Form einer Suspension hergestellt wird, die die zu analysierende Zelle enthält und auf einem aus CaF₂ hergestellten Substrat angeordnet wird, das mit einer Schicht beschichtet ist, die die Zelladhäsion ermöglicht.

5. Verfahren nach Anspruch 1, **dadurch gekennzeichnet, dass** in Schritt a) die die analysierte Zelle enthaltende Probe 15 Minuten lang mit einer warmen Lösung von 100 nM MitoBADY in Hanks' Balanced Salt Solution (HBSS), die Ca²⁺ - und Mg²⁺-Ionen enthält, inkubiert wird, mit einem DMSO-Gehalt von nicht mehr als 0,001% (v/v).

6. Verfahren nach Anspruch 1, **dadurch gekennzeichnet, dass** in Schritt b) die die analysierte Zelle enthaltende Probe während der Messung von Raman-Spektren in eine warme HBSS-Lösung, die Ca²⁺- und Mg²⁺-Ionen enthält, eingetaucht wird.

7. Verfahren nach Anspruch 1, **dadurch gekennzeichnet, dass** in Schritt b) die Messung des Raman-Spektrums an lebenden Zellen durchgeführt wird.

8. Verfahren nach Anspruch 1, **dadurch gekennzeichnet, dass** in Schritt b) das Raman-Spektrum mit einem Raman-Spektrometer mit Laseranregung bei 532 nm aufgezeichnet wird, das bevorzugt mit einem Wasserimmersionsobjektiv ausgestattet ist.

9. Verfahren nach Anspruch 1, **dadurch gekennzeichnet, dass** in Schritt (b) das Raman-Spektrum von mindestens 2 verschiedenen Punkten der analysierten Zelle aufgezeichnet wird, die Banden zeigen, die in Raman-Spektren für Cytochrom charakteristisch sind, bevorzugt Banden bei 750, 1130, 1315 und 1585 cm⁻¹, wobei die Positionen der Banden zwischen den verwendeten Spektrometern und Beugungsgittern unterschiedlich sein können.

10. Verfahren nach Anspruch 1, **dadurch gekennzeichnet, dass** in Schritt b) das Raman-Spektrum im Fingerprint-Bereich (500 - 3050 cm⁻¹) aufgezeichnet wird, und dann die integrierten Intensitäten der Banden bei 750, 2220 und 2850 cm⁻¹ ± 3 cm⁻¹ berechnet werden.

11. Verfahren nach Anspruch 1, **dadurch gekennzeichnet, dass** in Schritt c) zusätzlich das Vorhandensein der Zelle mit einem neutrophil-ähnlichen Phänotyp durch eine Erhöhung der Intensität der Raman-Banden bei 2222 cm⁻¹ und eine Erhöhung des Wertes des Verhältnisses der integrierten Intensitäten der Banden 750/(750+2222) (A-Verhältnis) nachgewiesen wird, bevorzugt über 1,24.

## Revendications

1. Procédé d'identification d'une cellule de type neutrophile obtenue à partir d'une cellule de leucémie promyélocytique, **caractérisé en ce que** :
a) la cellule analysée est mise en contact in vitro avec un rapporteur MitoBADY, puis
b) son spectre Raman est enregistré et les intensités intégrées des bandes de 750, 2220 et 2850 cm⁻¹ ± 3 cm⁻¹ sont déterminées,
c) les valeurs des rapports A et A/B sont calculées, où :
A est le rapport des intensités intégrées de bande 750 / (750 + 2222), tandis que
B est le rapport des intensités intégrées de bande 2850 / (2850 + 2222),
où une cellule est considérée comme différenciée en neutrophile lorsque la valeur du rapport A/B est supérieure à 1,12,
où, de préférence, la cellule de leucémie promyélocytique est humaine, en particulier une cellule de la lignée HL-60.

2. Procédé selon la revendication 1, **caractérisé en ce que** la cellule avec un phénotype de type neutrophile est un résultat d'une différenciation intentionnelle ou d'un processus spontané se produisant pendant une culture.

3. Procédé selon la revendication 1 ou la revendication 2, **caractérisé en ce que** la cellule subit une différenciation en neutrophile par incubation avec de l'acide tout-trans rétinoïque (ATRA) ou du diméthylsulfoxyde (DMSO).

4. Procédé selon la revendication 1, **caractérisé en ce que**, dans l'étape a), un échantillon est préparé sous la forme d'une suspension contenant la cellule à analyser et placé sur un substrat constitué de CaF₂ revêtu d'une couche permettant l'adhésion cellulaire.

5. Procédé selon la revendication 1, **caractérisé en ce que**, dans l'étape a), l'échantillon contenant la cellule analysée est incubé pendant 15 min avec une solution chaude de 100 nM de MitoBADY dans une Solution saline équilibrée de Hanks (HBSS) contenant des ions Ca²⁺ et Mg²⁺, avec une teneur en DMSO ne dépassant pas 0,001 % (v/v).

6. Procédé selon la revendication 1, **caractérisé en ce que**, dans l'étape b), l'échantillon contenant la cellule analysée pendant la mesure des spectres Raman est immergé dans une solution HBSS chaude contenant des ions Ca²⁺ et Mg²⁺.

7. Procédé selon la revendication 1, **caractérisé en ce que**, dans l'étape b), la mesure du spectre Raman est effectuée dans des cellules vivantes.

8. Procédé selon la revendication 1, **caractérisé en ce que**, dans l'étape b), le spectre Raman est enregistré avec un spectromètre Raman avec excitation laser à 532 nm, équipé de préférence d'un objectif à immersion dans l'eau.

9. Procédé selon la revendication 1, **caractérisé en ce que**, dans l'étape b), le spectre Raman est enregistré à partir d'au moins 2 points différents de la cellule analysée montrant des bandes caractéristiques d'un cytochrome dans les spectres Raman, de préférence des bandes de 750, 1130, 1315 et 1585 cm⁻¹, tandis que les positions des bandes peuvent différer entre les spectromètres et les réseaux de diffraction utilisés.

10. Procédé selon la revendication 1, **caractérisé en ce que**, dans l'étape b), le spectre Raman est enregistré dans la région d'empreinte digitale (500 - 3050 cm⁻¹), et ensuite, les intensités intégrées des bandes de 750, 2220 et 2850 cm⁻¹ ± 3 cm⁻¹ sont calculées.

11. Procédé selon la revendication 1, **caractérisé en ce que**, dans l'étape c), de plus, la présence de la cellule avec un phénotype de type neutrophile est mise en évidence par une augmentation de l'intensité des bandes Raman à 2222 cm⁻¹ et une augmentation de la valeur du rapport des intensités intégrées des bandes 750 / (750 + 2222) (rapport A), de préférence supérieure à 1,24.
